# EUROPÄISCHE PATENTSCHRIFT

(11) **EP 3 902 592 B1**
(45) Veröffentlichungstag und Bekanntmachung des Hinweises auf die Patenterteilung: **28.01.2026**
(21) Anmeldenummer: 19835424.3
(22) Anmeldetag: 27.12.2019
(51) Int. Cl.: F16K 17/04, F16K 17/06, F16K 17/12, A61M 27/00

(54) **VENTIL ZUM DAUERHAFTEN IMPLANTIEREN, INSBESONDERE ZUR BEHANDLUNG DER NORMALDRUCK-HYDROZEPHALUS-ERKRANKUNG**
VALVE FOR PERMANENT IMPLANTATION, IN PARTICULAR FOR TREATMENT OF NORMAL PRESSURE HYDROCEPHALUS
VALVE IMPLANTABLE DURABLEMENT, EN PARTICULIER POUR LE TRAITEMENT DE L'HYDROCÉPHALIE À PRESSION NORMALE

(30) Priorität: 28.12.2018 DE 102018133691
(43) Veröffentlichungstag der Anmeldung: 03.11.2021
(73) Patentinhaber: Universität Bern, 3012 Bern (CH); Spiegelberg, Andreas, 8810 Horgen (CH)
(72) Erfinder: BECK, Jürgen, 3065 Bolligen (CH); SPIEGELBERG, Andreas, 8810 Horgen (CH)
(74) Vertreter: Schulz Junghans Patentanwälte PartGmbB
(86) Internationale Anmeldenummer: PCT/EP2019/087124
(87) Internationale Veröffentlichungsnummer: WO 2020/136278

(56) Entgegenhaltungen:
- EP-A1- 2 221 083
- EP-B1- 2 221 083
- US-A- 3 985 140
- US-A- 5 336 166
- US-A- 5 368 556
- US-A1- 2014 316 325

## Beschreibung

Die vorliegende Erfindung betrifft ein implantierbares Ventil, insbesondere zum dauerhaften Implantieren zur Behandlung der Hydrozephalus-Erkrankung.

Für die Behandlung von Patienten, bei denen ein Ungleichgewicht zwischen der Bildung von Hirnwasser und der Rückresorption von Hirnwasser besteht, den sogenannten Hydrozephaluspatienten, werden Drainageeinrichtungen verwandt, welche das Hirnwasser (Liquor cerebrospinalis) aus den Liquorräumen des Gehirnes in eine andere Körperhöhle ableiten. Vollständig implantierbare Drainageeinrichtungen bestehen üblicherweise aus einem Ventrikelkatheter, der mit seiner Spitze in der Hirnkammer des Patienten platziert wird und unter der Haut zu einem Ventil geführt wird, dem Ventil, das den Rückstrom von Hirnwasser verhindert und eine Überdruckfunktion hat, und einem peripheren Katheter, der den Auslass des Ventils mit einem Hohlraum des Körpers, zum Beispiel der Bauchhöhle verbindet. Derartige Drainageeinrichtungen werden auch als Shuntsysteme, die Ventile als Shuntventile bezeichnet. Nachdem erste und einfache Ableitsysteme lediglich aus einem Schlauch bestanden, werden heute Shuntventile verwendet, um eine Überdrainage von Liquor zu verhindern. Die Liquor-Überdrainage, eine bekannte und gefürchtete Komplikation von Drainagesystemen, kann unerwünschte Folgen wie Konzentrationsstörungen, Kopfschmerzen und intrakranielle Blutungen bis hin zum Tode nach sich ziehen.

Ein gemeines Funktionsprinzip dieser Überdruckventile ist, dass sie, zunächst geschlossen, erst oberhalb eines bestimmten Drucks, des Ansprechdrucks, öffnen und erst dann Liquor drainieren. So soll eine Überdrainage verhindert werden.

Für derartige Einsatzzwecke geeignete Ventile sind entweder mit einem festen Ansprechdruck, der für den Patienten passend individuell auswählbar ist, ausgestattet, oder sie sind einstellbar.

Ferner sind Shuntventile bekannt, bei denen der Ansprechdruck abhängig von der Körperposition zwischen einem höheren Wert in der aufrechten Position und einem niedrigeren Wert in der liegenden Position automatisch umgeschaltet wird, oder stufenlos angepasst wird. Für diese Systeme sind verschiedene Bezeichnungen üblich, sie werden vorliegend gesamthaft als schwerkraftkompensierte Ventile bezeichnet.

Shuntsysteme wurden ursprünglich zur Behandlung solcher Hydrozephaluserkrankungen entwickelt, die mit der Entwicklung eines deutlich pathologischen Überdruckes einhergehen. Dabei handelt es sich um die große Gruppe der frühkindlichen Hydrozephalus-Patienten und um solche in höherem Lebensalter erworbene Erkrankungen, bei denen der Abfluss- und Resorptionsvorgang gestört ist, oder bei denen eine der Verbindungen zwischen den verschiedenen Liquorräumen verengt oder verschlossen ist.

Eine besondere Gruppe der Hydrozephaluspatienten sind die sogenannten Normaldruck-Hydrozephalus-Patienten (normal pressure hydrocephalus, NPH). Bei diesen ist der mittlere Druck im Hirnwasser nicht pathologisch erhöht. Es kommt aber zu wellenförmigen Erhöhungen des Druckes, insbesondere im Schlaf und im Liegen. Die Behandlung dieser Patientengruppe mit Drainageeinrichtungen bzw. Shuntsystemen ist mit besonderen Schwierigkeiten behaftet: Weil der mittlere Druck nicht dauerhaft erhöht ist, ist die Einstellung oder Wahl der Ansprechschwelle des Shuntventiles besonders kritisch. Schon wenig zu hohe Ansprechschwellen führen zu mangelhaften Behandlungsergebnissen. Andererseits kommt es bei diesen Patienten, die erwachsen und mobil sind, in der aufrechten Körperposition oft zu hydrostatisch bedingter Überdrainage, was zu ernsthaften Komplikationen führt. Die Verwendung von schwerkraftkompensierten Systemen bringt Verbesserungen, aber die Charakteristik der Schwerkraftkompensation ist genau wie die oben genannte Ansprechschwelle sehr kritisch und muss in engen Grenzen den Erfordernissen des einzelnen Patienten angepasst sein.

Es ist zur Behandlung des NPH versucht worden, das Hirnwasser im Bereich der Lendenwirbelsäule aus dem subduralen Liquorraum unter der Dura mater (harte Hirnhaut) in den epiduralen Bereich über der Dura mater abzuleiten. Die Shuntsysteme, die bei diesen Versuchen eingesetzt wurden, unterscheiden sich wesentlich von den üblichen ventrikulo-peritonealen Shuntsystemen. Fulcher und Enomoto (O. H. Fulcher and F. Enomoto, "Some simple methods of treating communicating hydrocephalus.," in Surgical forum, 1956, vol. 8, pp. 516-521) verwendeten ein offenes Stück Kunststoffschlauch, das mit Widerhaken versehen war, damit es am Platz blieb. Quincke (H. Quincke, "Die Lumbalpunktion des Hydrocephalus," Berliner Klin. Wochenschrift, vol. 28, no. 38, pp. 929-933) punktierte den Subduralraum und schlitzte gleichzeitig die Dura, so dass es zu einer bleibenden Drainageöffnung kam. Glenn (US020060224102, US020060224101, US000007513883) beschreibt ein System, welches einen Einlass unterhalb der harten Hirnhaut, einen damit verbundenen Durchflussregler und einen damit verbundenen Auslass zur Platzierung im epiduralen Raum aufweist.

Weiterhin sind allgemein Ventile aus der EP0268520A2, EP2253352A1, JPS5230089A, US4945947A, US2010056980A1, EP0414649, US4682 625, US2977 980, FR 2 685 206 bzw. US 5 336 166 bekannt.

Ferner beschreibt die EP2221083A1 ein Ventil, das zum Einsatz in einer Leitung zum Führen einer Flüssigkeit dient, wobei das Ventil zwischen einer vorgegebenen Flüssigkeitsdruck-Untergrenze und einer vorgegebenen Flüssigkeitsdruck-Obergrenze geöffnet ist und beim Unterschreiten der Flüssigkeitsdruck-Untergrenze einerseits und beim Überschreiten der Flüssigkeitsdruck-Obergrenze andererseits zumindest zeitweilig und zumindest weitgehend geschlossen ist.

Weiterhin beschreibt die US5336166A eine implantierbare Drainagevorrichtung für die Behandlung von Hydrocephalus. Die Vorrichtung funktioniert wie eine Kombination aus drei Ventilen, die ein erstes Druckregulierungsventil in Reihe mit einer Baugruppe mit einem Durchflussregulierungsventil parallel zu einem zweiten Druckregulierungsventil umfassen.

Hiervon ausgehend liegt der vorliegenden Erfindung die Aufgabe zugrunde, ein verbessertes implantierbares Ventil bereitzustellen, das insbesondere zur Shuntbehandlung des NPH unter besonderer Beachtung der besonderen Erfordernisse dieser Patienten geeignet ist.

Diese Aufgabe wird durch ein Ventil mit den Merkmalen des Anspruchs 1 sowie durch eine Drainageeinrichtung mit den Merkmalen des Anspruchs 34 gelöst. Vorteilhafte Ausführungsformen dieser Erfindungsaspekte sind in den entsprechenden Unteransprüchen angegeben.

Gemäß Anspruch 1 wird ein implantierbares Ventil für eine Drainageeinrichtung zum Ableiten von Hirnwasser offenbart, mit:
- einem entlang einer Ventilachse erstreckten Ventilgehäuse, das einen Einlass und einen Auslass sowie einen vom Ventilgehäuse umgebenen Innenraum aufweist,
- einer im Innenraum angeordneten Ventilkörperanordnung, die z.B. in Richtung der Ventilachse beweglich im Innenraum angeordnet ist,
- einem ersten Ventilsitz, wobei die Ventilkörperanordnung dazu konfiguriert ist, zum Verschließen einer Strömungsverbindung zwischen dem Einlass und dem Innenraum des Ventilgehäuses abdichtend an dem ersten Ventilsitz anzuliegen,
- einem zweiten Ventilsitz, wobei die Ventilkörperanordnung dazu konfiguriert ist, zum Verschließen einer Strömungsverbindung zwischen dem Auslass und dem Innenraum des Ventilgehäuses abdichtend an dem zweiten Ventilsitz anzuliegen,
- einer im Innenraum angeordneten Federvorrichtung, die eine Federkraft auf die Ventilkörperanordnung in Richtung auf den ersten Ventilsitz ausübt,
wobei die Federkraft bei einer horizontalen Lage des Ventilgehäuses, bei der die Ventilachse horizontal verläuft, größer ist als eine horizontale Komponente der Gewichtskraft der Ventilkörperanordnung, und wobei die Gewichtskraft der Ventilkörperanordnung in der vertikalen Lage des Ventilgehäuses größer als die Federkraft ist.

Insbesondere ist die Ventilkörperanordnung in Richtung der Ventilachse zwischen den beiden Ventilsitzen angeordnet, wobei das Ventil Flüssigkeit bzw. Hirnwasser durchlässt (vom Einlass zum Auslass), wenn die Ventilkörperanordnung an beiden Ventilsitzen nicht abdichtend anliegt. Dieser Zustand des Ventils wird als offen bezeichnet. Liegt hingegen die Ventilkörperanordnung an einem der Ventilsitze an, ist das Ventil geschlossen und verhindert einen Durchfluss von Liquor/Hirnwasser. Überraschenderweise wird von den behandelnden Ärzten festgestellt, dass bei NPH-Patienten die einmalige Drainage kleiner Volumina ausreicht, um einen über Tage anhaltenden Behandlungserfolg zu erzielen.

Das erfindungsgemäße Ventil kann dies sicherstellen, da es dazu konfiguriert ist, in aufrechter Körperposition (oder bei vertikaler Ventilachse) vollständig geschlossen zu sein (z.B. in dem die Schwerkraft den Ventilkörper gegen den zweiten Ventilsitz drückt) und weiterhin dazu konfiguriert ist, in liegender Körperposition (oder bei horizontaler Ventilachse) eine Drainage bei einem (insbesondere niedrigen) Ansprechdruck zu erlauben (d.h. bei dem Ansprechdruck offen ist), jedoch insbesondere einen Rückstrom (im Sinne eines Rückschlagventiles) verhindert.

Gemäß einer bevorzugten Ausführungsform der Erfindung ist vorgesehen, dass die Gewichtskraft der Ventilkörperanordnung bei einer vertikalen Lage des Ventilgehäuses, bei der die Ventilachse vertikal verläuft und der erste Ventilsitz oberhalb des zweiten Ventilsitzes angeordnet ist, in Richtung auf den zweiten Ventilsitz wirkt.

Gemäß einer weiteren bevorzugten Ausführungsform der Erfindung ist vorgesehen, dass der zweite Ventilsitz bei einer geneigten Lage des Ventilgehäuses, bei der die Ventilachse einen Winkel mit der Vertikalen im Bereich von 0° bis 40° einschließt, durch die Ventilkörperanordnung verschlossen ist, wobei, wenn der Winkel 40° überschreitet, die parallel zur Ventilachse verlaufende Komponente der Gewichtskraft die Summe aus Druckkraft und Federkraft unterschreitet, wobei die Ventilkörperanordnung sich (insbesondere sprunghaft) in Richtung auf den ersten Ventilsitz bewegt und diesen (insbesondere übergangslos) verschließt.

Gemäß einer weiteren bevorzugten Ausführungsform der Erfindung ist vorgesehen, dass der erste Ventilsitz bei einer geneigten Lage des Ventilgehäuses, bei der die Ventilachse einen Winkel mit der Horizontalen im Bereich von 0° bis 40° einschließt, durch die Ventilkörperanordnung verschlossen ist. Bei diesem Übergang wirken gleichzeitig die (parallel zur Ventilachse) zunehmende Gewichtkraft und die durch die Lageänderung zunehmende Druckkraft auf die Ventilkörperanordnung, so dass sich eine steile Kennlinie ergibt. Mit zunehmender Steigung der Zunahme der Druckkraft wird der Übergangsbereich also immer kleiner, wodurch ein problematisch großer Übergangsbereich nicht vorkommt.

Insbesondere funktioniert damit die vorliegende Erfindung nun erstmals gegenüber den eingangs dargelegten Vorgehensweisen nach einem umgekehrten Prinzip: die Liquordrainage bzw. Hirnwasserdrainage ist nur bei niedrigen Differenzdrücken (in der horizontalen Lage) möglich. Bei höheren Differenzdrücken, die nur in der vertikalen Lage auftreten, ist das Ventil verschlossen.

Diese Funktionsweise ist besonders effektiv für die Vermeidung einer Überdrainage. Denn gerade dann, wenn ein hoher Druckgradient besteht (z.B. im Stehen - hier addieren sich ca. 30cm bis 60 cm hydrostatische Wassersäule (beim Erwachsenen), vom Kopf zur Ableitstelle (spinaler Epiduralraum oder Bauchhöhle) hinzu), wird bei den klassischen Ventilsystemen besonders viel Liquor drainiert und es besteht die Gefahr einer Überdrainage.

Die erfindungsgemäße Lösung weist also die Vorteile auf, dass unphysiologisch hohe Drainagemengen vermieden werden können (das Ventil ist nur offen bzw. im Durchflusszustand bei einem niedrigeren Druck).

Das erfindungsgemäße Ventil bildet weiterhin einen zuverlässiger Schalter (Ventil offen / geschlossen) unter Ausnutzung einer großen Druckdifferenz von 30 cm bis 60 cm Wassersäule (hydrostatische Druckdifferenz vom Übergang Liegen zum Stehen beim Erwachsenen bzw. Übergang horizontale Lage zu vertikale Lage des Ventilgehäuses)

Weiterhin sind mit Vorteil die Druckverhältnisse im Ableitraum (z.B. spinaler Subarachnoidalraum) und Drainageraum (z.B. spinaler Epiduralraum oder Bauchhöhle) auf annähernd gleichem Niveau. Hier kommt es nur zu geringen Druckgradienten. Dieses Implantationsprinzip senkt ebenfalls die Gefahr einer gefährlichen Überdrainage.

Insgesamt bewirkt die Funktionsweise des erfindungsgemäßen Ventils eine effiziente Drainage bei einem deutlich besseren Schutz vor einer Überdrainage.

Gemäß einer Ausführungsform der Erfindung ist vorgesehen, dass die Ventilkörperanordnung durch den ersten Ventilkörper gebildet ist.

Gemäß einer alternativen Ausführungsform der Erfindung ist vorgesehen, dass die Ventilkörperanordnung einen zweiten Ventilkörper aufweist, wobei der erste Ventilkörper zur Anlage am ersten Ventilsitz konfiguriert ist, und wobei der zweite Ventilkörper zur Anlage am zweiten Ventilsitz konfiguriert ist und/oder schwerer ist als der erste Ventilkörper.

Gemäß einer weiteren Ausführungsform der Erfindung ist vorgesehen, dass die Ventilkörperanordnung (neben dem ersten Ventilkörper) einen zweiten Ventilkörper und einen dritten Ventilkörper aufweist, wobei der erste Ventilkörper zur Anlage am ersten Ventilsitz konfiguriert ist, und wobei der dritte Ventilkörper zur Anlage am zweiten Ventilsitz konfiguriert ist, und wobei der zweite Ventilkörper schwerer ist als der erste Ventilkörper und/oder als der dritte Ventilkörper. Weiterhin kann das Ventil auch mehrere zweite Ventilkörper aufweisen, die vorzugsweise zwischen dem ersten und dem dritten Ventilkörper angeordnet sind.

Weiterhin ist hierbei gemäß einer Ausführungsform vorzugsweise vorgesehen, dass der zweite Ventilkörper zwischen dem ersten und dem dritten Ventilkörper angeordnet ist und insbesondere mit dem ersten und dem dritten Ventilkörper in Kontakt steht.

Weiterhin ist gemäß einer bevorzugten Ausführungsform vorgesehen, dass der jeweilige Ventilkörper (d.h. der erste und/oder der zweite und/oder der dritte Ventilkörper) zumindest abschnittsweise zylinderförmig ausgebildet ist, wobei insbesondere der jeweilige Ventilkörper zylinderförmig ausgebildet sein kann.

Gemäß einer weiteren Ausführungsform der Erfindung ist vorgesehen, dass der jeweilige Ventilkörper (oder einer der Ventilkörper, insbesondere der erste und/oder der zweite und/oder der dritte Ventilkörper) kugelförmig ausgebildet ist.

Weiterhin ist gemäß einer bevorzugten Ausführungsform der Erfindung vorgesehen, dass der jeweilige Ventilkörper (oder einer der Ventilkörper, insbesondere der erste und/oder der zweite und/oder der dritte Ventilkörper) zylinderförmig ausgebildet ist und eine erste ebene Endfläche aufweist, die zur Anlage an den ersten Ventilsitz ausgebildet ist oder zur Anlage an einen benachbarten Ventilkörper, sowie eine der ersten Endfläche abgewandte zweite Endfläche, die zur Anlage am zweiten Ventilsitz ausgebildet ist oder zur Anlage an einen benachbarten Ventilkörper.

Weiterhin ist gemäß einer bevorzugten alternativen Ausführungsform der Erfindung vorgesehen, dass der jeweilige Ventilkörper (oder einer der Ventilkörper, insbesondere der erste und/oder der zweite und/oder der dritte Ventilkörper) einen zylinderförmig ausgebildeten mittleren Abschnitt aufweist, der zwischen einem ersten Endabschnitt und einem zweiten Endabschnitt angeordnet ist (der mittlere Abschnitt verbindet die beiden Endabschnitte insbesondere einstückig miteinander), wobei der erste Endabschnitt zur Anlage an den ersten Ventilsitz ausgebildet ist oder zur Anlage an einen benachbarten Ventilkörper, und wobei der zweite Endabschnitt zur Anlage am zweiten Ventilsitz ausgebildet ist oder zur Anlage an einen benachbarten Ventilkörper. Die beiden Endabschnitte können hierbei jeweils gekrümmt oder halbkugelförmig oder kegelstumpfförmig oder kegelförmig ausgebildet sein.

Weiterhin ist gemäß einer Ausführungsform der Erfindung vorgesehen, dass ein Durchmesser des zweiten Ventilkörpers größer ist als ein Durchmesser des ersten Ventilkörpers und/oder als ein Durchmesser des dritten Ventilkörpers der Ventilkörperanordnung.

Weiterhin ist gemäß einer bevorzugten Ausführungsform der Erfindung vorgesehen, dass der jeweilige Ventilkörper der Ventilkörperanordnung (oder einer der Ventilkörper, insbesondere der erste und/oder der zweite und/oder der dritte Ventilkörper) aus einem Material besteht, das eine Dichte von mehr als 10 g/cm³ aufweist.

Weiterhin ist gemäß einer bevorzugten Ausführungsform der Erfindung vorgesehen, dass der jeweilige Ventilkörper (oder einer der Ventilkörper, insbesondere der erste und/oder der zweite und/oder der dritte Ventilkörper) und/oder das Ventilgehäuse und/oder die Feder aus einem biokompatiblen bzw. körperverträglichen Material besteht oder mit einem solchem Material beschichtet ist. Ein biokompatibles Material ist insbesondere ein Material, das keinen negativen Einfluss auf Lebewesen, insbesondere Menschen, in seiner Umgebung hat.

Gemäß einer bevorzugten Ausführungsform der Erfindung ist vorgesehen, dass der jeweilige Ventilkörper (oder einer der Ventilkörper, insbesondere der erste und/oder der zweite und/oder der dritte Ventilkörper) aus einem der folgenden Stoffe besteht oder einen der folgenden Stoffe aufweist, Wolfram, eine Wolframlegierung, Wolframkarbid, Diwolframkarbid, einer Legierung aufweisend Wolframkarbid und/oder Diwolframkarbid.

Gemäß einer bevorzugten Ausführungsform der Erfindung ist vorgesehen, dass der Ventilkörper aus einem der folgenden Stoffe besteht oder einen der folgenden Stoffe aufweist, Silber, Gold, Platin, Tantal oder, einer Legierung einen der Stoffe Silber, Gold, Platin, Tantal.

Weiterhin ist gemäß einer Ausführungsform der Erfindung vorgesehen, dass der Einlass des Ventils dem Auslass des Ventils in Richtung der Ventilachse gegenüberliegt, und/oder dass der zweite Ventilsitz dem ersten Ventilsitz in Richtung der Ventilachse gegenüberliegt.

Weiterhin ist gemäß einer Ausführungsform der Erfindung vorgesehen, dass die Federvorrichtung eine erste Feder aufweist. Insbesondere kann die Federvorrichtung durch die erste Feder gebildet sein.

Weiterhin ist gemäß einer Ausführungsform der Erfindung vorgesehen, dass das Ventilgehäuse einen in Richtung auf den ersten Ventilsitz in den Innenraum abstehenden Vorsprung aufweist.

Gemäß einer Ausführungsform ist dabei vorgesehen, dass der Vorsprung einen Ringspalt mit einer in Umfangsrichtung des Ventilgehäuses umlaufenden Wandung des Ventilgehäuses bzw. einer umlaufenden Innenseite dieser Wandung ausbildet.

Gemäß einer Ausführungsform verbindet diese umlaufende Wandung einen erste Wand des Ventilgehäuses mit einer in Richtung der Ventilachse gegenüberliegenden zweiten Wand des Ventilgehäuses verbindet, wobei gemäß einer Ausführungsform der Auslass an der ersten Wand und der Einlass an der zweiten Wand vorgesehen ist.

Weiterhin ist gemäß einer Ausführungsform der Erfindung vorgesehen, dass der zweite Ventilsitz an einem dem ersten Ventilsitz bzw. der zweiten Wand zugewandten Endabschnitt des Vorsprungs vorgesehen ist. Der Vorsprung umgibt insbesondere eine vorzugsweise längserstreckte Durchgangsöffnung, die den Auslass des Ventils bildet. Der zweite Ventilsitz wird z.B. durch eine umlaufende Stirnseite des Vorsprungs gebildet, der jene Durchgangsöffnung am Ende des Vorsprungs umrandet. Weiterhin kann der zweite Ventilsitz auch durch einen umlaufenden Abschnitt des Vorsprungs (z.B. in Form einer Stufe) gebildet sein.

Weiterhin ist gemäß einer Ausführungsform der Erfindung vorgesehen, dass die erste Feder den Vorsprung umgreift (und dabei insbesondere in dem Ringspalt angeordnet ist). Die erste Feder kann insbesondere als Schraubenfeder ausgebildet sein.

Weiterhin ist gemäß einer Ausführungsform der Erfindung vorgesehen, dass sich die erste Feder mit einem ersten Endabschnitt der ersten Feder an einer Innenseite der ersten Wand des Ventilgehäuses abstützt, von der der Vorsprung in den Innenraum des Ventilgehäuses absteht.

Weiterhin ist gemäß einer Ausführungsform der Erfindung vorgesehen, dass die Ventilanordnung vorzugsweise aus dem mindestens einen ersten Ventilkörper besteht, wobei die erste Feder mit einem zweiten Endabschnitt den mindestens eine ersten Ventilkörper kontaktiert und die besagte Federkraft in Richtung auf den ersten Ventilsitz auf den ersten Ventilkörper ausübt.

Weiterhin ist gemäß einer alternativen Ausführungsform der Erfindung vorgesehen, dass die Ventilanordnung aus dem mindestens einen ersten Ventilkörper und dem zweiten Ventilkörper besteht bzw. diese Ventilkörper aufweist, wobei die erste Feder mit einem zweiten Endabschnitt den zweiten Ventilkörper kontaktiert und die Federkraft in Richtung auf den ersten Ventilsitz auf den zweiten Ventilkörper ausübt, wobei der zweite Ventilkörper dazu konfiguriert ist, die Federkraft auf den ersten Ventilkörper zu übertragen.

Weiterhin ist gemäß einer Ausführungsform der Erfindung vorgesehen, dass die Ventilanordnung aus dem mindestens einen ersten Ventilkörper, dem zweiten Ventilkörper und dem dritten Ventilkörper besteht bzw. diese Ventilkörper aufweist, wobei die erste Feder mit einem zweiten Endabschnitt den zweiten Ventilkörper kontaktiert und einen ersten Teil der Federkraft in Richtung auf den ersten Ventilsitz auf den zweiten Ventilkörper ausübt, wobei der zweite Ventilkörper dazu konfiguriert ist, den ersten Teil der Federkraft auf den ersten Ventilkörper zu übertragen.

Weiterhin ist gemäß einer Ausführungsform hierbei vorgesehen, dass die Federvorrichtung eine zweite Feder aufweist (wobei insbesondere die Federvorrichtung durch die erste und die zweite Feder gebildet ist), wobei sich die zweite Feder mit einem ersten Endabschnitt an einer Innenseite des Ventilgehäuses abstützt, und wobei die zweite Feder mit einem zweiten Endabschnitt den dritten Ventilkörper kontaktiert und einen zweiten Teil der Federkraft in Richtung auf den ersten Ventilsitz auf den dritten Ventilkörper ausübt, wobei der dritte Ventilkörper dazu konfiguriert ist, den zweiten Teil der Federkraft über den zweiten Ventilkörper auf den ersten Ventilkörper zu übertragen.

Weiterhin ist gemäß einer Ausführungsform der Erfindung vorgesehen, dass der Auslass durch eine längs erstreckte Durchgangsöffnung des Ventilgehäuses gebildet ist, wobei die Durchgangsöffnung eine Stufe aufweist. Insbesondere ist die Durchgangsöffnung als Stufenbohrung ausgeführt.

Weiterhin ist gemäß einer Ausführungsform der Erfindung vorgesehen, dass sich die zweite Feder mit dem ersten Endabschnitt an der Stufe abstützt.

Weiterhin ist gemäß einer Ausführungsform der Erfindung vorgesehen, dass die zweite Feder in der Durchgangsöffnung angeordnet ist.

Auch bei der zweiten Feder kann es sich gemäß einer Ausführungsform um eine Schraubenfeder handeln.

Weiterhin kann die zweite Feder koaxial zur ersten Feder angeordnet sein. Die erste Feder kann die zweite Feder umgreifen.

Der Einlass des Ventils ist insbesondere als eine Durchgangsöffnung der zweiten Wand des Ventilgehäuses ausgebildet,

Der erste Ventilsitz wird gemäß einer Ausführungsform durch einen umlaufenden Randbereich des Einlasses bzw. der entsprechenden Durchgangsöffnung gebildet, wobei jener Randbereich den Einlass bzw. die Durchgangsöffnung umrandet.

Das erfindungsgemäße Ventil ist des Weiteren nicht auf eine lineare Bewegung des Ventilkörpers beschränkt. So ist gemäß einer alternativen Ausführungsform der Erfindung vorgesehen, dass der Ventilkörper schwenkbar am Ventilgehäuse im Innenraum gelagert ist, so dass der Ventilkörper zwischen dem ersten und dem zweiten Ventilsitz hin und her schwenkbar ist, wobei die Feder (insbesondere Drehfeder) hier ebenfalls versucht, den Ventilkörper zum ersten Ventilsitz zu bewegen.

Weiterhin kann sich der Ventilkörper grundsätzlich aus mehreren, insbesondere separaten Körpern zusammensetzen. So kann z.B. gemäß einer Ausführungsform vorgesehen sein, dass der Ventilkörper einen ersten Körper und einen zweiten Körper aufweist, wobei der erste Körper zur Anlage am ersten Ventilsitz konfiguriert ist, und wobei der zweite Körper zur Anlage am zweiten Ventilsitz konfiguriert ist und/oder schwerer ist als der erste Körper. Ein Großteil des Gewichts des Ventilkörpers kann hierbei durch den zweiten Körper gegeben sein, während der erste Körper z.B. die Abdichtung am ersten Ventilsitz übernimmt.

Weiterhin kann gemäß einer Ausführungsform der Erfindung vorgesehen sein, dass der Ventilkörper einen ersten Körper, einen oder mehrere zweite Körper und einen dritten Körper aufweist, wobei der erste Körper zur Anlage am ersten Ventilsitz konfiguriert ist, und wobei der dritte Körper zur Anlage am zweiten Ventilsitz konfiguriert ist, und wobei der oder die zweiten Körper schwerer ist bzw. sind als der erste Körper und/oder der dritte Körper.

Gemäß einer weiteren Ausführungsform des erfindungsgemäßen Ventils ist vorgesehen, dass die Masse der Ventilkörperanordnung und die Federvorrichtung bzw. deren Federkraft so gewählt sind, dass die Schwerkraft auf die Ventilkörperanordnung die Lage der Ventilkörperanordnung relativ zu dem ersten und dem zweiten Ventilsitz nicht beeinflusst, so dass sich insbesondere die besagte Lage bezüglich der Ventilsitze nicht ändert, wenn sich eine Raumlage der Ventilachse des Ventils ändert.

Gemäß einem weiteren Aspekt der vorliegenden Erfindung wird eine Drainageeinrichtung mit den Merkmalen des Anspruchs 34 offenbart. Danach weist die erfindungsgemäße Drainageeinrichtung zur Drainage von Hirnwasser zumindest auf:
- ein erfindungsgemäßes Ventil,
- einen ersten Katheter (insbesondere Ventrikelkatheter), der einen in einer Hirnkammer oder dem spinalen Liquorraum eines Patienten positionierbaren Endabschnitt aufweist und mit dem Einlass des Ventils in Strömungsverbindung steht, so dass Hirnwasser über den ersten Endabschnitt in den ersten Katheter eintreten kann, und
- einen zweiten Katheter, der mit dem Auslass des Ventils in Strömungsverbindung steht und einen in einem Hohlraum (z.B. Bauchhöhle, Epiduralraum) des Körpers des Patienten positionierbaren Endabschnitt aufweist, so dass Hirnwasser aus dem zweiten Katheter über den Endabschnitt in den Hohlraum austreten kann.

Insbesondere weist also der Endabschnitt des ersten Katheters zumindest eine Einlassöffnung auf, über die Hirnwasser in den ersten Katheter eintreten kann. Ebenso weist der Endabschnitt des zweiten Katheters zumindest eine Auslassöffnung auf, über die Hirnwasser aus dem zweiten Katheter austreten kann.

Die erfindungsgemäße Drainageeinrichtung ist insbesondere zur Behandlung der Normaldruck-Hydrozephalus-Erkrankung geeignet. Der Einlass des Ventils ist dabei über den ersten Katheter mit dem Liquorraum oder Subduralraum des Patienten im Bereich der Lendenwirbelsäule (dem spinalen Liquorraum) verbindbar. Der Auslass des Ventils ist demgegenüber über den zweiten Katheter mit dem Epiduralraum der Lendenwirbelsäule des Patienten verbindbar.

Weitere Ausführungsformen, Merkmale und Vorteile der vorliegenden Erfindung sollen nachfolgend bei der Beschreibung von Ausführungsbeispielen mit Bezug auf die Figuren erläutert werden. Es zeigen:
- Fig. 1: eine Ausführungsform eines erfindungsgemäßen Ventils in vertikaler Lage;
- Fig. 2: eine Ausführungsform eines erfindungsgemäßen Ventils in horizontaler Lage;
- Fig. 3: verschiedene Formen eines Ventilkörpers eines erfindungsgemäßen Ventils;
- Fig. 4: eine Ausführungsform eines erfindungsgemäßen Ventils mit einem aus zwei Körpern zusammengesetzten Ventilkörper;
- Fig. 5: eine Ausführungsform eines erfindungsgemäßen Ventils mit einem aus drei Körpern zusammengesetzten Ventilkörper;
- Fig. 6: eine Ausführungsform eines erfindungsgemäßen Ventils mit schwenkbaren Ventilkörper,
- Fig. 7: eine Ausführungsform eines erfindungsgemäßen Ventils, wobei hier die Lage des Ventilkörpers unabhängig von der Raumlage des Ventils ist; und
- Fig. 8: eine Modifikation der in der Fig. 7 gezeigten Ausführungsform.
- Fig. 9: eine erfindungsgemäße Drainageeinrichtung mit einem erfindungsgemäßen Ventil.

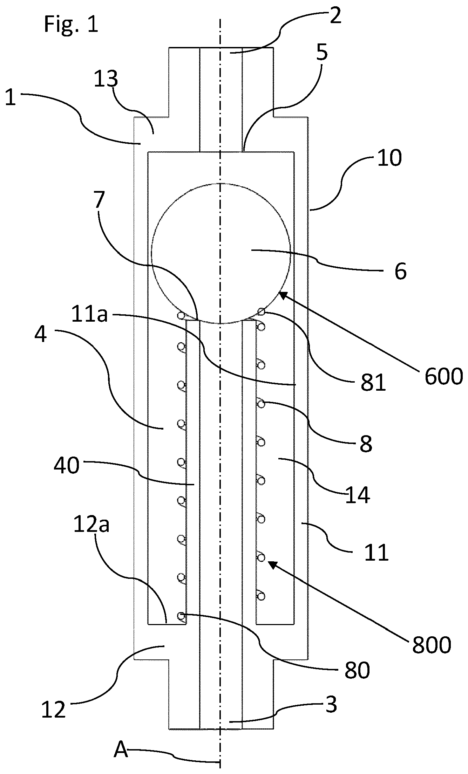

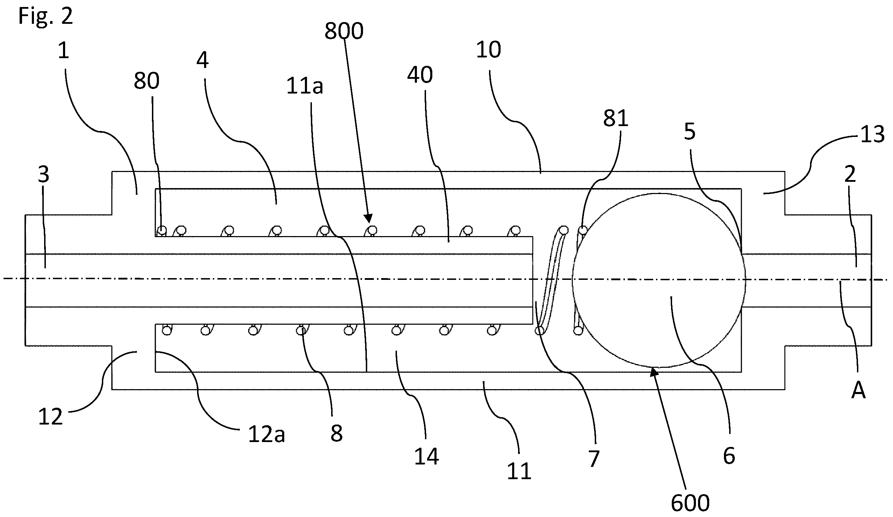

Figur 1 zeigt im Zusammenhang mit Figur 2 ein erfindungsgemäßes Ventil 1. Das Ventil 1 weist ein Ventilgehäuse 10 auf, welches einen Einlass 2 und einen Auslass 3 und einen Innenraum 4 aufweist. Das Ventilgehäuse 10 erstreckt sich dabei entlang einer Ventilachse A, wobei der Einlass 2 und der Auslass 3 einander in Richtung der Ventilachse A gegenüberliegen.

Zwischen dem Einlass 2 und dem Innenraum 4 befindet sich ein erster Ventilsitz 5, der von einer Ventilkörperanordnung 600, die hier aus einem Ventilkörper 6 besteht, verschlossen werden kann. Weiterhin befindet sich zwischen dem Innenraum 4 und dem Auslass 3 ein zweiter Ventilsitz 7, der ebenfalls von dem Ventilkörper 6 verschlossen werden kann.

Das Ventilgehäuse 10 weist eine in Umfangsrichtung U des Ventilgehäuses 10 umlaufende Wandung 11 auf, die eine umlaufende dem Innenraum 4 zugewandte Innenseite 11a aufweist. Die umlaufende Wandung 11 verbindet eine erste und eine zweite Wand 12, 13 des Ventilgehäuses 10 miteinander, wobei insbesondere der Auslass 3 an der ersten Wand 12 und der Einlass 2 an der zweiten Wand 13 vorgesehen ist.

Weiterhin ist im Innenraum 4 insbesondere ein Vorsprung 40 vorgesehen, der von einer Innenseite 12a der ersten Wand 12 in Richtung auf den ersten Ventilsitz 5 bzw. die zweite Wand 13 absteht. Der Vorsprung 40 bildet dabei einen Ringspalt 14 mit der Innenseite 11a der umlaufenden Wandung 11 des Ventilgehäuses 10.

Der zweite Ventilsitz 7 ist insbesondere an einem dem ersten Ventilsitz 5 bzw. der zweiten Wand 13 zugewandten Ende des Vorsprungs 40 vorgesehen. Der Vorsprung 40 umgibt insbesondere eine längs erstreckte Durchgangsöffnung 3, die den Auslass 3 des Ventils 1 bildet. Der zweite Ventilsitz 7 wird insbesondere durch eine umlaufende Stirnseite 7 des Vorsprungs 40 gebildet, der jene Durchgangsöffnung bzw. Auslass 3 am Ende des Vorsprungs 40 umrandet.

Weiterhin weist das Ventil 1 bevorzugt eine Federvorrichtung 800 auf, die hier aus einer Feder 8 besteht, die den Ventilkörper 6 gegen den ersten Ventilsitz 5 drückt. Die Feder 8, die Abmaße und das Material des Ventilkörpers 6 sind so dimensioniert, dass in vertikaler Position, die in Figur 1 gezeigt ist, die Gewichtskraft des Ventilkörpers 6 die Federkraft der Feder 8 übersteigt, so dass der Ventilkörper 6 den ersten Ventilsitz 5 freigibt und den zweiten Ventilsitz 7 verschließt. In dieser vertikalen Position des Ventilgehäuses 10 (die Ventilachse A ist hierbei vertikal ausgerichtet) ist das Ventil 1 also für Hirnwasser, das am Einlass 2 anliegt, geschlossen. In horizontaler Position (Ventilachse A verläuft horizontal), die in der Figur 2 gezeigt ist, überwiegt die Federkraft der Feder 8, und der zweite Ventilsitz 7 ist freigegeben. Der Ventilkörper liegt am ersten Ventilsitz 5 an und verschließt das Ventil 1. Überschreitet der Druck des Hirnwassers einen Schwellenwert, gibt der Ventilkörper 6 den ersten Ventilsitz frei und kommt jedoch nicht zur Anlage an den zweiten Ventilsitz 7, so dass Hirnwasser über das Ventil 1 abgeführt werden kann.

Die Kombination aus Ventilkörper 6, Feder 8 und erstem Ventilsitz 5 bildet in dieser horizontalen Position also ein Überdruckventil mit Rückschlagfunktion. Das Ventil 1 ist also für Hirnwasser geöffnet, wenn ein Schwellwert überschritten wird. Es ist gegen Rückstrom gesichert.

Insbesondere ist vorgesehen, dass die Feder 8 den Vorsprung 40 umgreift (und dabei insbesondere in dem Ringspalt 14 angeordnet ist). Die Feder 8 kann gemäß Figuren 1 und 2 insbesondere als Schraubenfeder ausgebildet sein. Hierbei kann sich die Feder 8 an einer Innenseite 12a der ersten Wand 12 des Ventilgehäuses 10 abstützen, von der der Vorsprung 40 in den Innenraum 4 des Ventilgehäuses 10 absteht.

In einer besonders bevorzugten Ausführungsform der Erfindung weist das Ventilgehäuse 10 einen kreisförmigen Querschnitt (senkrecht zur Ventilachse A) wobei die umlaufende Wandung 11 einen äußeren Durchmesser aufweist, der gemäß einem Beispiel der Erfindung 6,5 mm beträgt, wobei der Durchmesser des Innenraumes 4 bzw. der innere Durchmesser der Wandung 11 5,5 mm. Der Durchmesser der Ventilsitze 5, 7 beträgt in dem Beispiel 1,2 mm.

Der Weg des Ventilkörpers 6 in Richtung der Ventilachse A von Ventilsitz 5 zu Ventilsitz 7 beträgt beispielsweise 1 mm. Der Ventilkörper 6 ist insbesondere als eine Kugel ausgebildet, die in dem besagten Beispiel einen Durchmesser von 5 mm aufweist und insbesondere aus Wolframkarbid mit einer Dichte von 16,63 g/cm³ besteht. Das Gewicht der Kugel 6 beträgt damit 1,088 g. Die Gewichtskraft in Wasser beträgt 0,01023 N (Gewicht abzüglich Auftrieb). Die Feder 8 (insbesondere Schraubenfeder) hat in dem Beispiel einen Drahtdurchmesser von 0,1 mm, eine entspannte Länge von 10 mm, einen Innendurchmesser von 2,5 mm und - bei 10 Windungen - eine Federkonstante von 0,005 N/mm. In der horizontalen Lage hat die Feder 8 eine vorgespannte Länge von 9,6 mm. Die Federkraft beträgt damit 0,002 N. Um in der horizontalen Lage die Kugel 6 gegen die Kraft, die durch das Kippmoment verursacht wird, welches durch die Gewichtskraft entsteht, in den ersten Ventilsitz 5 zu drücken ist eine Federkraft von mindestens 0,0019 N erforderlich, was damit erreicht ist. In der Vertikalen drückt die Gewichtskraft der Kugel 6 die Feder 8 um einen Weg von maximal 2 mm auf minimal 8 mm zusammen, so dass die konstruktiv gewählte komprimierte Länge von z.B. 8,6 mm sicher erreicht werden kann. Der maximale intrakranielle Druck von 50 mmHg = 7000 Pa drückt die Kugel 6 mit einer Kraft von 0,005 N in Richtung auf den zweiten Ventilsitz 7. Die Gewichtskraft der Kugel von 0,01023 N abzüglich der Schließkraft der Feder von 0,002 N beträgt 0,00823 N. Die Kugel 6 wird somit durch den intrakraniellen Druck und durch die Gewichtskraft der Kugel vom ersten Ventilsitz 5 gelöst und wird gegen den zweiten Ventilsitz gedrückt und verschließt diesen.

In einer weiteren bevorzugten Ausführung der Erfindung hat die Kugel einen Durchmesser von 6,5 mm und besteht aus Stahl, und das Gehäuse hat einen Innendurchmesser von 7 mm und einen Außendurchmesser von 8 mm. Die Masse der Feder und die Durchmesser der Ventilsitze sind wie in dem oben angegebenen Beispiel ausgestaltet.

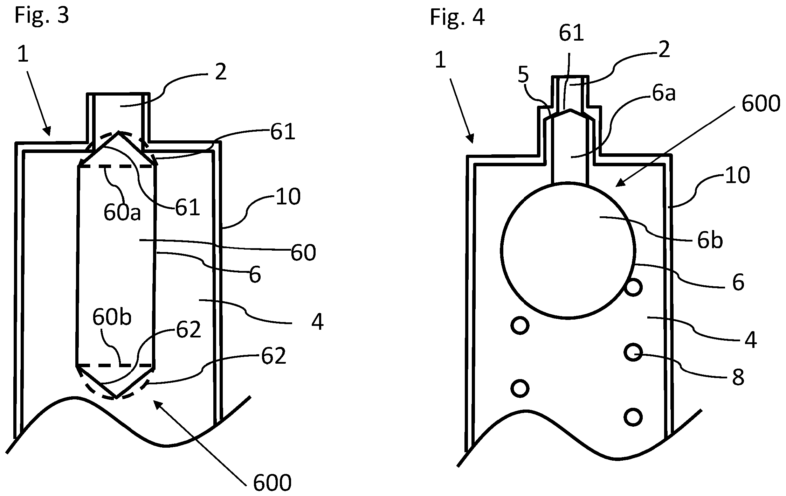

Gemäß Figur 3 kann der Ventilkörper 6 verschiedenen Ausgestaltungen aufweisen und kann insbesondere von einer kugelförmigen Geometrie abweichen.

So kann gemäß Figur 3 der Ventilkörper 6 zylinderförmig ausgebildet sein und eine ebene erste Endfläche 60a aufweisen (gestrichelte Linie), die zur Anlage an den ersten Ventilsitz 5 ausgebildet ist, sowie eine der ersten Endfläche 60a abgewandte ebene zweite Endfläche 60b (gestrichelte Linie), die zur Anlage am zweiten Ventilsitz 7 ausgebildet ist.

Alternativ hierzu kann der Ventilkörper 6 gemäß Figur 3 einen zylinderförmig ausgebildeten mittleren Abschnitt 60 aufweisen, der zwischen einem ersten Endabschnitt 61 (gestrichelte Linie) und einem zweiten Endabschnitt 62 (gestrichelte Linie) des Ventilkörpers 6 angeordnet ist, wobei der erste Endabschnitt 61 zur Anlage an den ersten Ventilsitz 5 ausgebildet ist, und wobei der zweite Endabschnitt 62 zur Anlage am zweiten Ventilsitz 7 ausgebildet ist. Hierbei können die beiden Endabschnitte 61, 62 z.B. gekrümmt, halbkugelförmig, kegelstumpfförmig oder kegelförmig ausgebildet sein

Weiterhin zeigt Fig. 4 eine Ausführungsform eines erfindungsgemäßen Ventils 1, bei der die Ventilkörperanordnung 600 einen ersten Ventilkörper 6a und einen separaten zweiten Ventilkörper 6b aufweist, wobei der erste (z.B. abschnittsweise zylinderförmig ausgestaltete) Ventilkörper 6a zur Anlage am ersten Ventilsitz 5 konfiguriert ist, und wobei der zweite (z.B. kugelförmig ausgestaltete) Ventilkörper 6b zur Anlage am zweiten Ventilsitz 7 konfiguriert ist und/oder schwerer ausgebildet ist als der erste Körper 6a.

Sinngemäss sind auch Ausführungsformen des erfindungsgemäßen Ventils 1 möglich, bei der die Ventilkörperanordnung 600 einen ersten Ventilkörper 6a, einen oder mehrere zweite Ventilkörper 6b und einen dritten Ventilkörper 6c aufweist, wobei der erste (z.B. kugelförmig oder abschnittsweise zylinderförmig ausgestaltete) Ventilkörper 6a zur Anlage am ersten Ventilsitz 5 konfiguriert ist, und wobei der dritte (z.B. kugelförmig oder abschnittsweise zylinderförmig ausgestaltete) Ventilkörper 6c zur Anlage am zweiten Ventilsitz 7 konfiguriert ist, und wobei der eine oder mehrere zweite Ventilkörper 6b schwerer ausgebildet ist bzw. sind als der erste Ventilkörper 4 6a und/oder der dritte Ventilkörper 6c.

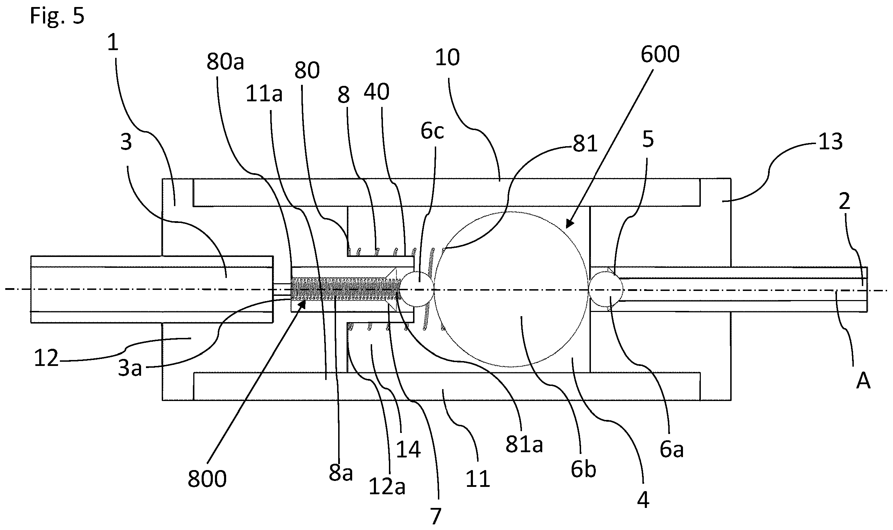

Eine solche Ausführungsform eines erfindungsgemäßen Ventils 1 mit einer aus drei Ventilkörpern 6a, 6b, 6c gebildeten Ventilkörperanordnung 600 ist in der Fig. 5 gezeigt. Hierbei sind die Ventilkörper 6a, 6b, 6c jeweils kugelförmig ausgebildet (andere Formen sind auch denkbar) und entlang der Ventilachse A nebeneinander angeordnet, wobei der mittlere zweite Ventilkörper 6b einen größeren Durchmesser aufweist als die beiden anderen Ventilkörper 6a, 6c, wobei der zweite Ventilkörper 6b insbesondere schwerer ist als der erste Ventilkörper 6a sowie schwerer als der dritte Ventilkörper 6c.

Ähnlich der in den Figuren 1 und 2 gezeigten Konfiguration ist vorzugsweise auch hier vorgesehen, dass das Ventilgehäuse 10 einen in Richtung auf den ersten Ventilsitz 5 in den Innenraum 4 Ventilgehäuses 10 abstehenden Vorsprung 40 aufweist, wobei der Vorsprung 40 einen Ringspalt 14 mit einer umlaufenden Innenseite 11a des Ventilgehäuses 10 ausbildet. Der erste Ventilsitz 5 liegt dem Vorsprung 40 in Richtung der Ventilachse A gegenüber und wird durch einen umlaufenden Randbereich des Einlasses 2 des Ventils ausgebildet, der z.B. konusförmig ausgestaltet sein kann. Demgegenüber ist der zweite Ventilsitz 7 an einem dem ersten Ventilsitz 5 zugewandten Endabschnitt des Vorsprungs 40 vorgesehen und kann als ein umlaufender Abschnitt (z.B. konusförmige Stufe) einer im Vorsprung 40 angeordneten Durchgangsöffnung 3 ausgebildet sein, die den Auslass 3 des Ventils 1 bildet.

Die drei Ventilkörper 6a, 6b, 6c sind entlang der Ventilachse A zwischen den beiden Ventilsitzen 5, 7 angeordnet, wobei der erste Ventilkörper 6a zur Anlage am ersten Ventilsitz 5 und der dritte Ventilkörper zur Anlage am zweiten Ventilsitz 7 konfiguriert ist.

Gemäß Fig. 5 weist das Ventil 1 weiterhin eine Federvorrichtung 800 auf, die aus einer ersten und einer zweiten Feder 8, 8a besteht, wobei die erste Feder 8 vorzugsweise eine Schraubenfeder ist, die den Vorsprung 40 umgreift. Dabei ist vorgesehen, dass sich die erste Feder 8 mit einem ersten Endabschnitt 80 an einer Innenseite 12a des Ventilgehäuses 10 abstützt, von der der Vorsprung 40 in den Innenraum 4 des Ventilgehäuses 10 absteht.

Im Unterschied zu den Figuren 1 und 2 ist hier nun vorgesehen, dass die erste Feder 8 mit einem zweiten Endabschnitt 81 den zweiten Ventilkörper 6b kontaktiert und einen ersten Teil einer Federkraft, die durch die Federvorrichtung 800 auf die Ventilkörperanordnung 600 ausgeübt werden soll, in Richtung auf den ersten Ventilsitz 5 auf den zweiten Ventilkörper 6b ausübt, wobei der zweite Ventilkörper 6b diesen ersten Teil der Federkraft auf den ersten Ventilkörper 6a überträgt.

Weiterhin weist die Federvorrichtung 800 im Unterscheid zu den Figuren 1 und 2 eine zweite Feder 8a auf, wobei sich die zweite Feder 8a mit einem ersten Endabschnitt 80a an einer Stufe 3a der Durchgangsöffnung 3 abstützt, in der die zweite Feder 8a angeordnet ist. Die zweite Feder 8a ist ebenfalls vorzugsweise als Schraubenfeder ausgeführt und weist einen geringeren Durchmesser auf als die erste Feder 8. Die zweite Feder 8a kontaktiert nun mit einem zweiten Endabschnitt 81a den dritten Ventilkörper 6b und über dabei einen zweiten Teil der besagten Federkraft in Richtung auf den ersten Ventilsitz 5 auf den dritten Ventilkörper 6c aus, wobei dieser den zweiten Teil der Federkraft über den zweiten Ventilkörper 6b auf den ersten Ventilkörper 6a überträgt.

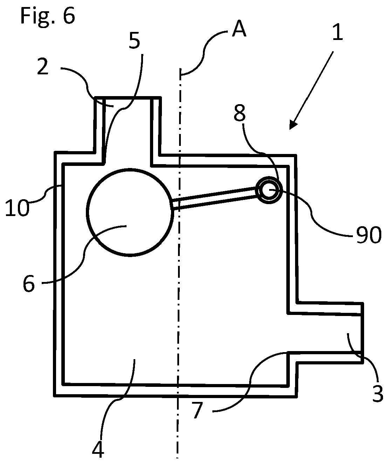

Die Figur 5 zeigt das Ventil in liegender Position (horizontale Ventilachse A), wobei die gesamte durch die beiden Federn 8, 8a bereitgestellte Federkraft den ersten Ventilkörper gegen den ersten Ventilsitz 5 drückt, so dass das Ventil 1 geschlossen ist. Diese Bauform hat den Vorteil, dass der zweite Ventilsitz bei einer geneigten Lage des Ventilgehäuses, bei der die Ventilachse einen Winkel mit der Vertikalen im Bereich von 0° bis 40° einschließt, durch die Ventilkörperanordnung verschlossen ist. Weiterhin zeigt Fig. 6 eine Ausführungsform eines erfindungsgemäßen Ventils 1, bei der der Ventilkörper 6 schwenkbar am Ventilgehäuse 10 im Innenraum 4 gelagert ist (z.B. über ein Drehlager 90) so dass der Ventilkörper 6 zwischen dem ersten und dem zweiten Ventilsitz 5, 7 hin und her schwenkbar ist. Hierbei kann die Feder 8, die versucht, den Ventilkörper 6 zum ersten Ventilsitz zu bewegen bzw. zu verschwenken, als Drehfeder 8 ausgebildet sein.

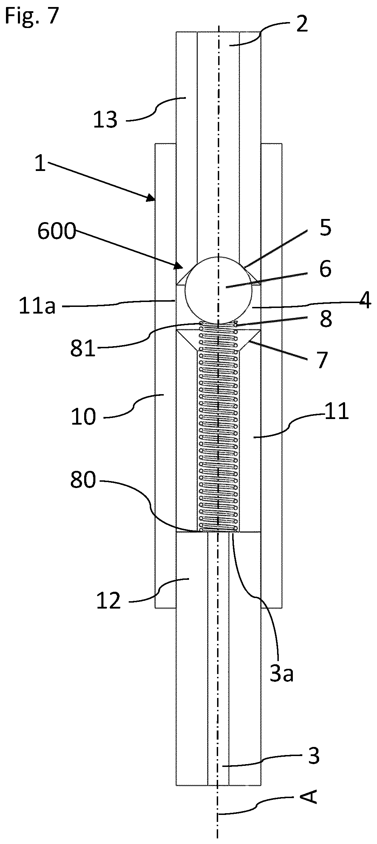

Die Figur 7 zeigt eine weitere Ausführungsform eines erfindungsgemäßen Ventils. Das Ventil 1 weist vorzugsweise ein Ventilgehäuse 10 auf, welches einen Einlass 2 und einen Auslass 3 sowie einen Innenraum 4 aufweist. Das Ventilgehäuse 10 erstreckt sich dabei entlang einer Ventilachse A, wobei der Einlass 2 und der Auslass 3 einander in Richtung der Ventilachse A gegenüberliegen.

Zwischen dem Einlass 2 und dem Innenraum 4 befindet sich einer erster Ventilsitz 5, der von einer Ventilkörperanordnung 600, die hier aus dem insbesondere kugelförmigen Ventilkörper 6 besteht, verschlossen werden kann. Weiterhin befindet sich zwischen dem Innenraum 4 und dem Auslass 3 ein zweiter Ventilsitz 7, der ebenfalls von dem Ventilkörper 6 verschlossen werden kann.

Das Ventilgehäuse 10 weist eine in Umfangsrichtung des Ventilgehäuses 10 umlaufende Wandung 11 auf, die eine umlaufende dem Innenraum 4 zugewandte Innenseite 11a aufweist. Die umlaufende Wandung 11 verbindet eine erste und eine zweite Wand 12, 13 des Ventilgehäuses 10 miteinander, wobei insbesondere der Auslass 3 an der ersten Wand 12 in Form einer längserstreckten Durchgangsöffnung 3 vorgesehen ist, und wobei insbesondere der Einlass 2 an der zweiten Wand 13 in Form einer längserstreckten Durchgangsöffnung 2 vorgesehen ist.

Das Ventil 1 weist weiterhin eine Federvorrichtung 800 auf, die durch eine Feder 8 gebildet wird, wobei sich die Feder 8 mit einem ersten Endabschnitt 80 an einer Stufe 3a der den Auslass 3 bildenden Durchgangsöffnung 3 abstützt, in der die Feder 8 angeordnet ist. Die Feder 8 ist vorzugsweise als Schraubenfeder ausgeführt und kontaktiert mit einem zweiten Endabschnitt 81 den Ventilkörper 6 und übt eine Federkraft in Richtung auf den ersten Ventilsitz 5 auf den Ventilkörper 6 aus.

Bei der Ausführungsform gemäß Figur 7 ist nun vorgesehen, dass die Masse des Ventilkörpers 6 bei gegebener Federvorrichtung 800 bzw. 8 so gewählt ist, dass seine Lage relativ zu den Ventilsitzen 5 und 7 nicht durch die Schwerkraft auf den Ventilkörper 6 beeinflusst wird. Dementsprechend wird die Funktion unabhängig von der Position relativ zur Richtung der Schwerkraft. In Ruhelage, ohne Druckunterschied zwischen Einlass 2 und Auslass 3, wird der Ventilkörper 6 durch die Kraft der Feder 8 gegen den ersten Ventilsitz 5 gedrückt. In dieser Situation wirkt der Ventilkörper 6 zusammen mit dem Ventilsitz 5 als Rückschlagventil, so dass im Falle einer negativen Druckdifferenz zwischen Einlass 2 und Auslass 3 das Ventil 1 keine Durchströmung erlaubt. Wenn das Druckgefälle zwischen Einlass 2 und Auslass 3 positiv ist und so gross ist, dass die Kraft der Feder 8 überwunden wird, bewegt sich der Ventilkörper 6 vom ersten Ventilsitz 5 weg und das Ventil 1 wird durchströmt. Der Durchfluss nimmt dabei mit steigender Druckdifferenz zu. Wegen des Strömungswiderstandes wird der Ventilkörper 6 bei steigender Druckdifferenz in Richtung zum zweiten Ventilsitz 7 hin bewegt. Wenn ein bestimmter Grenzdruck erreicht wird, berührt der Ventilkörper 6 den zweiten Ventilsitz 7 und verschließt diesen. Das Ventil 1 ist nun verschlossen und erlaubt keine Durchströmung. Wenn die Druckdifferenz unter einen bestimmten zweiten Grenzdruck fällt, wird die Kraft der Feder 8 größer, als die Druckkraft, die den Ventilkörper 6 gegen den zweiten Ventilsitz 7 drückt, und das Ventil 1 öffnet wieder.

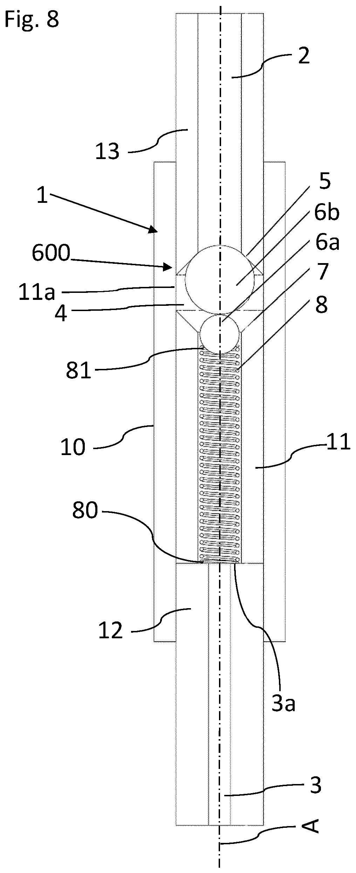

Figur 8 zeigt eine Modifikation der in der in Fig. 7 gezeigten Ausführungsform, wobei hier anstelle des Ventilkörpers 6 eine Ventilkörperanordnung 600 mit einem ersten Ventilkörper 6a und einem zweiten Ventilkörper 6b vorgesehen ist, wobei der erste Ventilkörper 6a dem Ventilkörper 6 der Figur 7 entspricht, und wobei im Unterschied zur Figur 7 die Kraft der Feder 8 nicht direkt auf den ersten Ventilkörper 6a wirkt, sondern über den zweiten Ventilkörper 6b, der in diesem Sinne einen Abstandskörper bildet, der den ersten Ventilkörper 6a kontaktiert. Der zweite Ventil- bzw. Abstandskörper 6b hat insbesondere die Funktion, ein Einklemmen einer obersten Windung 8b der Feder 8 zwischen dem zweiten Ventilsitz 7 und dem ersten Ventilkörper 6a zu verhindern.

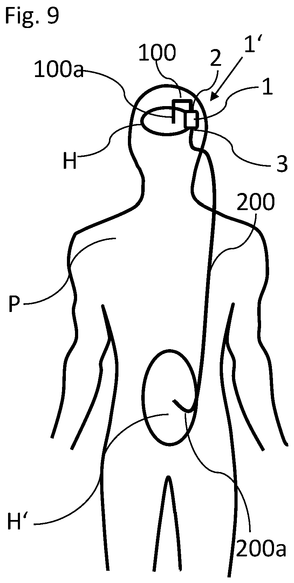

Figur 9 zeigt eine erfindungsgemäße Drainageeinrichtung 1' zur Drainage von Hirnwasser, die ein erfindungsgemäßes Ventil 1 aufweist, das nach einem der hierin beschriebenen Ausführungsbeispiele ausgeführt sein kann, sowie einen ersten Katheter 100, der einen in der Hirnkammer H oder im Liquorraum eines Patienten P anzuordnenden Endabschnitt 100a aufweist und mit dem Einlass 2 des Ventils 1 in Strömungsverbindung steht. Weiterhin weist die Einrichtung 1' einen zweiten Katheter 200 auf, der mit dem Auslass 3 des Ventils 1 in Strömungsverbindung steht und einen in einem Ableitort bzw. Hohlraum H' des Körpers des Patienten P anzuordnenden Endabschnitt 200a aufweist.

## Patentansprüche

1. Implantierbares Ventil (1) für eine Drainageeinrichtung (1') zum Ableiten von Hirnwasser, mit:
- einem entlang einer Ventilachse (A) erstreckten Ventilgehäuse (10), das einen Einlass (2) und einen Auslass (3) sowie einen vom Ventilgehäuse (10) umgebenen Innenraum (4) aufweist,
- einer im Innenraum (4) angeordneten Ventilkörperanordnung (600), die beweglich im Innenraum (4) angeordnet ist und zumindest einen ersten Ventilkörper (6, 6a, 6b, 6c) aufweist,
- einem ersten Ventilsitz (5), wobei die Ventilkörperanordnung (600) dazu konfiguriert ist, sich zum Verschließen einer Strömungsverbindung zwischen dem Einlass (2) und dem Innenraum (4) des Ventilgehäuses (10) an den ersten Ventilsitz (5) anzulegen,
- einem zweiten Ventilsitz (7), wobei die Ventilkörperanordnung (6) dazu konfiguriert ist, sich zum Verschließen einer Strömungsverbindung zwischen dem Auslass (3) und dem Innenraum (4) des Ventilgehäuses (10) an den zweiten Ventilsitz (7) anzulegen, und
- einer im Innenraum (4) angeordneten Federvorrichtung (800), die eine Federkraft auf die Ventilkörperanordnung (600) in Richtung auf den ersten Ventilsitz (5) ausübt,
wobei die Federkraft bei einer horizontalen Lage des Ventilgehäuses (10), bei der die Ventilachse (A) horizontal verläuft, größer ist als eine horizontale Komponente der Gewichtskraft der Ventilkörperanordnung (600), **dadurch gekennzeichnet, dass** die Gewichtskraft der Ventilkörperanordnung (600) in der vertikalen Lage des Ventilgehäuses (10) größer als die Federkraft ist.

2. Ventil nach Anspruch 1, **dadurch gekennzeichnet, dass** die Gewichtskraft der Ventilkörperanordnung (600) bei einer vertikalen Lage des Ventilgehäuses (10), bei der die Ventilachse (A) vertikal verläuft und der erste Ventilsitz (5) oberhalb des zweiten Ventilsitzes (7) angeordnet ist, in Richtung auf den zweiten Ventilsitz (7) wirkt.

3. Ventil nach einem der vorhergehenden Ansprüche, **dadurch gekennzeichnet, dass** die Ventilkörperanordnung (600) einen zweiten Ventilkörper (6b) aufweist, wobei der erste Ventilkörper (6a) zur Anlage am ersten Ventilsitz (5) konfiguriert ist, und wobei der zweite Ventilkörper (6b) zur Anlage am zweiten Ventilsitz (7) konfiguriert ist und/oder schwerer ist als der erste Ventilkörper (6a).

4. Ventil nach einem der Ansprüche 1 bis 2, **dadurch gekennzeichnet, dass** die Ventilkörperanordnung (6) einen zweiten Ventilkörper (6b) und einen dritten Ventilkörper (6c) aufweist, wobei der erste Ventilkörper (6a) zur Anlage am ersten Ventilsitz (5) konfiguriert ist, und wobei der dritte Ventilkörper (6c) zur Anlage am zweiten Ventilsitz (7) konfiguriert ist, und wobei der zweite Ventilkörper (6b) schwerer ist als der erste Ventilkörper (6a) und/oder der dritte Ventilkörper (6c).

5. Ventil nach Anspruch 4, **dadurch gekennzeichnet, dass** der zweite Ventilkörper (6b) zwischen dem ersten und dem dritten Ventilkörper (6a, 6c) angeordnet ist.

6. Ventil nach einem der Ansprüche 1 bis 5, **gekennzeichnet dadurch, dass** der jeweilige Ventilkörper (6, 6a, 6b, 6c) der Ventilkörperanordnung einen ersten Endabschnitt (61) und einen zweiten Endabschnitt (62) aufweist, wobei insbesondere der erste Endabschnitt (61) zur Anlage an den ersten Ventilsitz (5) oder zur Anlage an einen benachbarten Ventilkörper ausgebildet ist, und wobei insbesondere der zweite Endabschnitt (62) zur Anlage am zweiten Ventilsitz (7) oder zur Anlage an einen benachbarten Ventilkörper ausgebildet ist.

7. Ventil nach einem der Ansprüche 3 bis 6, **dadurch gekennzeichnet, dass** ein Durchmesser des zweiten Ventilkörpers (6b) größer ist als ein Durchmesser des ersten Ventilkörpers (6a) und/oder als ein Durchmesser des dritten Ventilkörpers (6b).

8. Ventil nach einem der vorhergehenden Ansprüche, **dadurch gekennzeichnet, dass** der Einlass (2) dem Auslass (3) in Richtung der Ventilachse (A) gegenüberliegt, und/oder dass der zweite Ventilsitz (7) dem ersten Ventilsitz (5) in Richtung der Ventilachse (A) gegenüberliegt, und/oder dass der jeweilige Ventilkörper (6, 6a, 6b, 6c) der Ventilkörperanordnung (600) in Richtung der Ventilachse (A) beweglich im Innenraum (4) angeordnet ist.

9. Ventil nach einem der vorhergehenden Ansprüche, **dadurch gekennzeichnet, dass** die Federvorrichtung (800) eine erste Feder (8) aufweist.

10. Ventil nach einem der vorhergehenden Ansprüche, **dadurch gekennzeichnet, dass** das Ventilgehäuse (10) einen in Richtung auf den ersten Ventilsitz (5) in den Innenraum (4) abstehenden Vorsprung (40) aufweist, wobei insbesondere der Vorsprung (40) einen Ringspalt (14) mit einer umlaufenden Innenseite (11a) des Ventilgehäuses (10) ausbildet, und wobei insbesondere der zweite Ventilsitz (7) an einem dem ersten Ventilsitz (5) zugewandten Endabschnitt des Vorsprungs (40) vorgesehen ist, und wobei insbesondere die erste Feder (8) den Vorsprung (40) umgreift.

11. Ventil nach Anspruch 10 und nach Anspruch 10, **dadurch gekennzeichnet, dass** sich die erste Feder (8) mit einem ersten Endabschnitt (80) an einer Innenseite (12a) des Ventilgehäuses (10) abstützt, wobei insbesondere von der Innenseite (12a) der Vorsprung (40) in den Innenraum (4) des Ventilgehäuses (10) absteht, wobei insbesondere die erste Feder (8) mit einem zweiten Endabschnitt (81) den mindestens eine ersten Ventilkörper (6) kontaktiert und die Federkraft in Richtung auf den ersten Ventilsitz (5) auf den ersten Ventilkörper (6) ausübt.

12. Ventilkörper nach Anspruch 11, **dadurch gekennzeichnet, dass** die erste Feder (8) mit einem zweiten Endabschnitt (81) den zweiten Ventilkörper (6b) kontaktiert und die Federkraft in Richtung auf den ersten Ventilsitz (5) auf den zweiten Ventilkörper (6b) ausübt, wobei der zweite Ventilkörper (6b) dazu konfiguriert ist, die Federkraft auf den ersten Ventilkörper (6a) zu übertragen, oder wobei die erste Feder (8) mit einem zweiten Endabschnitt (81) den zweiten Ventilkörper (6b) kontaktiert und einen ersten Teil der Federkraft in Richtung auf den ersten Ventilsitz (5) auf den zweiten Ventilkörper (6b) ausübt, wobei der zweite Ventilkörper (6b) dazu konfiguriert ist, den ersten Teil der Federkraft auf den ersten Ventilkörper (6a) zu übertragen.

13. Ventil nach Anspruch 12, **dadurch gekennzeichnet, dass** die Federvorrichtung (800) eine zweite Feder (8a) aufweist, wobei sich die zweite Feder (8a) mit einem ersten Endabschnitt (80a) an einer Innenseite (12a) des Ventilgehäuses (10) abstützt, und wobei die zweite Feder (8a) mit einem zweiten Endabschnitt (81a) den dritten Ventilkörper (6b) kontaktiert und einen zweiten Teil der Federkraft in Richtung auf den ersten Ventilsitz (5) auf den dritten Ventilkörper (6c) ausübt, wobei der dritte Ventilkörper (6c) dazu konfiguriert ist, den zweiten Teil der Federkraft über den zweiten Ventilkörper (6b) auf den ersten Ventilkörper (6a) zu übertragen.

14. Drainageeinrichtung (1') zur Drainage von Hirnwasser, mit:
- einem Ventil (1) nach einem der vorhergehenden Ansprüche,
- einem ersten Katheter (100), der einen in der Hirnkammer (H) eines Patienten (P) anordenbaren Endabschnitt (100a) aufweist und mit dem Einlass (2) des Ventils (1) in Strömungsverbindung steht, und
- einem zweiten Katheter (200), der mit dem Auslass (3) des Ventils (1) in Strömungsverbindung steht und einen in einem Hohlraum (H') des Körpers des Patienten (P) anordenbaren Endabschnitt (200a) aufweist.

## Claims

1. Implantable valve (1) for a drainage device (1') for draining cerebrospinal fluid, comprising:
- a valve housing (10) extending along a valve axis (A), which has an inlet (2) and an outlet (3) as well as an interior space (4) surrounded by the valve housing (10),
- a valve body assembly (600) arranged in the interior space (4), which is movably arranged in the interior space (4) and has at least a first valve body (6, 6a, 6b, 6c),
- a first valve seat (5), wherein the valve body assembly (600) is configured to abut against the first valve seat (5) to close a flow connection between the inlet (2) and the interior (4) of the valve housing (10),
- a second valve seat (7), wherein the valve body assembly (6) is configured to abut against the the second valve seat (7) to close a flow connection between the outlet (3) and the interior (4) of the valve housing (10), and
- a spring device (800) arranged in the interior (4), which exerts a spring force on the valve body assembly (600) in the direction of the first valve seat (5),
wherein the spring force is greater than a horizontal component of the weight of the valve body assembly (600) when the valve housing (10) is in a horizontal position in which the valve axis (A) extends horizontally, **characterized in that** the weight of the valve body assembly (600) in the vertical position of the valve housing (10) is greater than the spring force.

2. Valve according to claim 1, **characterized in that** the weight of the valve body assembly (600) acts in the direction of the second valve seat (7) when the valve housing (10) is in a vertical position in which the valve axis (A) runs vertically and the first valve seat (5) is arranged above the second valve seat (7).

3. Valve according to one of the preceding claims, **characterized in that** the valve body assembly (600) has a second valve body (6b), wherein the first valve body (6a) is configured to abut against the first valve seat (5), and wherein the second valve body (6b) is configured to abut against the second valve seat (7) and/or is heavier than the first valve body (6a).

4. Valve according to any one of claims 1 to 2, **characterized in that** the valve body assembly (6) comprises a second valve body (6b) and a third valve body (6c), wherein the first valve body (6a) is configured to abut against the first valve seat (5), and wherein the third valve body (6c) is configured to abut against the second valve seat (7), and wherein the second valve body (6b) is heavier than the first valve body (6a) and/or the third valve body (6c).

5. Valve according to claim 4, **characterized in that** the second valve body (6b) is arranged between the first and third valve bodies (6a, 6c).

6. Valve according to one of claims 1 to 5, **characterized in that** the respective valve body (6, 6a, 6b, 6c) of the valve body assembly has a first end section (61) and a second end section (62), wherein in particular the first end section (61) is designed to abut against the first valve seat (5) or to abut against an adjacent valve body, and wherein, in particular, the second end portion (62) is designed to abut against the second valve seat (7) or to abut against an adjacent valve body.

7. Valve according to one of claims 3 to 6, **characterized in that** a diameter of the second valve body (6b) is larger than a diameter of the first valve body (6a) and/or than a diameter of the third valve body (6c).

8. Valve according to one of the preceding claims, **characterized in that** the inlet (2) is opposite the outlet (3) in the direction of the valve axis (A), and/or that the second valve seat (7) is opposite the first valve seat (5) in the direction of the valve axis (A), and/or that the respective valve body (6, 6a, 6b, 6c) of the valve body assembly (600) is arranged in the interior space (4) so as to be movable in the direction of the valve axis (A).

9. Valve according to one of the preceding claims, **characterized in that** the spring device (800) has a first spring (8).

10. Valve according to one of the preceding claims, **characterized in that** the valve housing (10) has a projection (40) projecting into the interior space (4) in the direction of the first valve seat (5), wherein in particular the projection (40) forms an annular gap (14) with a circumferential inner side (11a) of the valve housing (10), and in particular the second valve seat (7) is provided at an end portion of the projection (40) facing the first valve seat (5), and in particular the first spring (8) engages around the projection (40).

11. Valve according to claim 10, **characterized in that** the first spring (8) is supported with a first end portion (80) on an inner side (12a) of the valve housing (10), wherein, in particular, the projection (40) projects from the inner side (12a) into the interior space (4) of the valve housing (10), wherein, in particular, the first spring (8) contacts the at least one first valve body (6) with a second end section (81) and exerts the spring force in the direction of the first valve seat (5) on the first valve body (6).

12. Valve according to claim 11, **characterized in that** the first spring (8) contacts the second valve body (6b) with a second end portion (81) and exerts the spring force in the direction of the first valve seat (5) on the second valve body (6b), wherein the second valve body (6b) is configured to transmit the spring force to the first valve body (6a), or wherein the first spring (8) contacts the second valve body (6b) with a second end portion (81) and exerts a first portion of the spring force in the direction of the first valve seat (5) on the second valve body (6b), wherein the second valve body (6b) is configured to transmit the first portion of the spring force to the first valve body (6a).

13. Valve according to claim 12, **characterized in that** the spring device (800) comprises a second spring (8a), wherein the second spring (8a) is supported with a first end portion (80a) on an inner side (12a) of the valve housing (10), and wherein the second spring (8a) contacts the third valve body (6c) with a second end portion (81a) and exerts a second portion of the spring force in the direction of the first valve seat (5) on the third valve body (6c), wherein the third valve body (6c) is configured to transmit the second portion of the spring force via the second valve body (6b) to the first valve body (6a).

14. Drainage device (1') for draining cerebrospinal fluid, comprising:
- a valve (1) according to one of the preceding claims,
- a first catheter (100) having an end section (100a) that can be positioned in the cerebral ventricle (H) of a patient (P) and is in fluid communication with the inlet (2) of the valve (1), and
- a second catheter (200) which is in fluid communication with the outlet (3) of the valve (1) and has an end section (200a) that can be positioned in a cavity (H') in the body of the patient (P).

## Revendications

1. Valve implantable (1) pour un dispositif de dérivation (1') pour l'évacuation de liquide cérébrospinal, avec :
- un logement de valve (10) étendu le long d'un axe de valve (A) qui présente une entrée (2) et une sortie (3) ainsi qu'un espace interne (4) entouré du logement de valve (10),
- un agencement de corps de valve (600) disposé dans l'espace interne (4) qui est disposé de manière mobile dans l'espace interne (4) et présente au moins un premier corps de valve (6, 6a, 6b, 6c),
- un premier siège de valve (5), dans laquelle l'agencement de corps de valve (600) est configuré pour s'appuyer sur le premier siège de valve (5) pour la fermeture d'une connexion d'écoulement entre l'entrée (2) et l'espace interne (4) du logement de valve (10),
- un second siège de valve (7), dans laquelle l'agencement de corps de valve (6) est configuré pour s'appuyer sur le second siège de valve (7) pour la fermeture d'une connexion d'écoulement entre la sortie (3) et l'espace interne (4) du logement de valve (10), et
- un dispositif de ressort (800) disposé dans l'espace interne (4) qui exerce une force élastique sur l'agencement de corps de valve (600) en direction du premier siège de valve (5),
dans laquelle la force élastique est supérieure dans le cas d'une position horizontale du logement de valve (10) dans laquelle l'axe de valve (A) s'étend horizontalement à une composante horizontale du poids de l'agencement de corps de valve (600), **caractérisée en ce que** le poids de l'agencement de corps de valve (600) est supérieur à la force élastique dans la position verticale du logement de valve (10).

2. Valve selon la revendication 1, **caractérisée en ce que** le poids de l'agencement de corps de valve (600) agit en direction du second siège de valve (7) dans le cas d'une position verticale du logement de valve (10) dans laquelle l'axe de valve (A) s'étend verticalement et le premier siège de valve (5) est disposé au-dessus du second siège de valve (7).

3. Valve selon une des revendications précédentes, **caractérisée en ce que** l'agencement de corps de valve (600) présente un deuxième corps de valve (6b), dans laquelle le premier corps de valve (6a) est configuré pour l'appui sur le premier siège de valve (5), et dans laquelle le deuxième corps de valve (6b) est configuré pour l'appui sur le second siège de valve (7) et/ou est plus lourd que le premier corps de valve (6a).

4. Valve selon une des revendications 1 à 2, **caractérisée en ce que** l'agencement de corps de valve (6) présente un deuxième corps de valve (6b) et un troisième corps de valve (6c), dans laquelle le premier corps de valve (6a) est configuré pour l'appui sur le premier siège de valve (5), et dans laquelle le troisième corps de valve (6c) est configuré pour l'appui sur le second siège de valve (7), et dans laquelle le deuxième corps de valve (6b) est plus lourd que le premier corps de valve (6a) et/ou le troisième corps de valve (6c).

5. Valve selon la revendication 4, **caractérisée en ce que** le deuxième corps de valve (6b) est disposé entre le premier et le troisième corps de valve (6a, 6c).

6. Valve selon une des revendications 1 à 5, **caractérisée en ce que** le corps de valve respectif (6, 6a, 6b, 6c) de l'agencement de corps de valve présente une première section d'extrémité (61) et une seconde section d'extrémité (62), dans laquelle la première section d'extrémité (61) est notamment réalisée pour l'appui sur le premier siège de valve (5) ou pour l'appui sur un corps de valve voisin, et dans laquelle la seconde section d'extrémité (62) est notamment réalisée pour l'appui sur le second siège de valve (7) ou pour l'appui sur un corps de valve voisin.

7. Valve selon une des revendications 3 à 6, **caractérisée en ce qu'**un diamètre du deuxième corps de valve (6b) est supérieur à un diamètre du premier corps de valve (6a) et/ou à un diamètre du troisième corps de valve (6c).

8. Valve selon une des revendications précédentes, **caractérisée en ce que** l'entrée (2) s'oppose à la sortie (3) en direction de l'axe de valve (A), et/ou que le second siège de valve (7) s'oppose au premier siège de valve (5) en direction de l'axe de valve (A), et/ou que le corps de valve respectif (6, 6a, 6b, 6c) de l'agencement de corps de valve (600) est disposé de manière mobile dans l'espace interne (4) en direction de l'axe de valve (A).

9. Valve selon une des revendications précédentes, **caractérisée en ce que** le dispositif de ressort (800) présente un premier ressort (8).

10. Valve selon une des revendications précédentes, **caractérisée en ce que** le logement de valve (10) présente une saillie (40) dépassant dans l'espace interne (4) en direction du premier siège de valve (5), dans laquelle la saillie (40) forme notamment une fente annulaire (14) avec un côté interne périphérique (11a) du logement de valve (10), et dans laquelle le second siège de valve (7) est notamment prévu sur une section d'extrémité de la saillie (40) tournée vers le premier siège de valve (5), et dans laquelle le premier ressort (8) englobe notamment la saillie (40).

11. Valve selon la revendication 10, **caractérisée en ce que** le premier ressort (8) s'appuie avec une première section d'extrémité (80) sur un côté interne (12a) du logement de valve (10), dans laquelle la saillie (40) dépasse notamment du côté interne (12a) dans l'espace interne (4) du logement de valve (10), dans laquelle le premier ressort (8) vient notamment en contact avec l'au moins un premier corps de valve (6) avec une seconde section d'extrémité (81) et la force élastique s'exerce sur le premier corps de valve (6) en direction du premier siège de valve (5).

12. Valve selon la revendication 11, **caractérisé en ce que** le premier ressort (8) vient en contact avec le deuxième corps de valve (6b) avec une seconde section d'extrémité (81) et la force élastique s'exerce sur le deuxième corps de valve (6b) en direction du premier siège de valve (5), dans lequel le deuxième corps de valve (6b) est configuré pour transmettre la force élastique au premier corps de valve (6a), ou dans lequel le premier ressort (8) vient en contact avec le deuxième corps de valve (6b) avec une seconde section d'extrémité (81) et une première partie de la force élastique s'exerce sur le deuxième corps de valve (6b) en direction du premier siège de valve (5), dans lequel le deuxième corps de valve (6b) est configuré pour transmettre la première partie de la force élastique au premier corps de valve (6a).

13. Valve selon la revendication 12, **caractérisée en ce que** le dispositif de ressort (800) présente un second ressort (8a), dans laquelle le second ressort (8a) s'appuie avec une première section d'extrémité (80a) sur un côté interne (12a) du logement de valve (10), et dans laquelle le second ressort (8a) vient en contact avec le troisième corps de valve (6c) avec une seconde section d'extrémité (81a) et une seconde partie de la force élastique s'exerce sur le troisième corps de valve (6c) en direction du premier siège de valve (5), dans laquelle le troisième corps de valve (6c) est configuré pour transmettre la seconde partie de la force élastique au premier corps de valve (6a) par le biais du deuxième corps de valve (6b).

14. Dispositif de dérivation (1') pour la dérivation de liquide cérébrospinal, avec :
- une valve (1) selon une des revendications précédentes,
- un premier cathéter (100) qui présente une section d'extrémité (100a) pouvant être disposée dans le ventricule (H) d'un patient (P) et est en connexion d'écoulement avec l'entrée (2) de la valve (1), et
- un second cathéter (200) qui est en connexion d'écoulement avec la sortie (3) de la valve (1) et présente une section d'extrémité (200a) pouvant être disposée dans un espace creux (H') du corps du patient (P).
